(19) Europäisches Patentamt
European Patent Office
Office européen des brevets

(11) Publication number: **0 115 969**
**A1**

(12) **EUROPEAN PATENT APPLICATION**

(21) Application number: **84300849.1**

(22) Date of filing: **10.02.84**

(51) Int. Cl.³: **C 07 D 499/00**, A 61 K 31/43
// C07F7/18, C07F9/65

(30) Priority: **10.02.83 JP 20777/83**

(43) Date of publication of application: **15.08.84**
**Bulletin 84/33**

(84) Designated Contracting States: **DE FR GB IT**

(71) Applicant: **SHIONOGI SEIYAKU KABUSHIKI KAISHA, 12, Dosho-machi, 3-chome Higashi-ku, Osaka-shi, Osaka 541 (JP)**

(72) Inventor: **Uyeo, Shoichiro, 407, Izutsuya-cho Yanagino-banba-rokkaku-sagaru, Nakagyo-ku Kyoto (JP)**

(74) Representative: **Bizley, Richard Edward et al, BOULT, WADE & TENNANT 27 Furnival Street, London EC4A 1PQ (GB)**

(54) **Penem compounds, their preparation, formulations containing the same and their use in combating bacteria.**

(57) 6-(1-Hydroxyethyl)-2-heterocyclic thiomethyl-2-penem-3-carboxylic acid derivatives represented by the following formula:

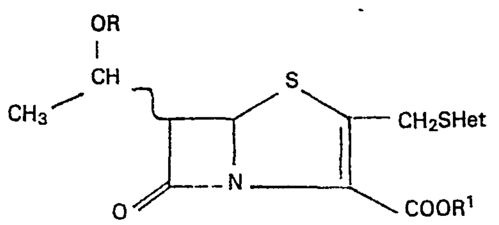
,

wherein R is hydrogen or a hydroxy-protecting group, $R^1$ is hydrogen, a light metal or a carboxy-protecting group and Het is a five-membered heterocyclic group, are useful as antibacterials against Gram-positive and Gram-negative bacteria.

EP 0 115 969 A1



BAD ORIGINAL

PENEM COMPOUNDS, THEIR PREPARATION, FORMULATIONS CONTAINING THE SAME AND THEIR USE IN COMBATING BACTERIA

This invention relates to new penem compounds.

More particularly, the present invention relates to 6-(-1-hydroxyethyl)-2-heterocyclic thiomethyl-2-penem-3-carboxylic acid derivatives represented by formula (I) below, their therapeutical use, processes for preparing them and pharmaceutical or veterinary formulations comprising them:

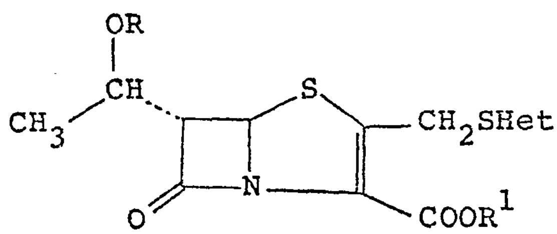

(I)

wherein R is hydrogen or a hydroxy-protecting group, $R^1$ is hydrogen, a light metal or a carboxy-protecting group, and Het is a five-membered heterocyclic group.

It has been found that the penem compounds (I) of the present invention are chemically stable and exhibit excellent antibiotic activity against various microorganisms, including those resistant to other different antibiotics.

In the above formula (I), R may preferably be hydrogen. Preferred hydroxy-protecting groups R include organic and inorganic acyl groups. Examples of the five-membered heterocyclic group represented by the symbol Het are pyrrole, furyl, thienyl, imidazole, isoxsazole, thiazole, isothiazole, triazole, oxadiazole, thiadiazole, tetrazole, thiatriazole, pyridine, pyrazine, pyrimidine, piperazine, triazine and tetrazine. These heterocyclic groups may be substituted by,

for example, alkyl, halogen, alkoxy, substituted alkyl, amino, hydroxy, carboxy, carbamoyl, acyloxy, phenyl, oxo or oxide groups.

Examples of the carboxyl-protecting group $R^1$ are those commonly employed in the penicillin and cephalosporin art in order to protect a carboxyl group without adversely effecting the other parts of the molecule. Illustrative of these protecting groups are aralkyl esters (e.g. esters of benzyl, methylbenzyl, dimethylbenzyl, methoxybenzyl, ethoxybenzyl, nitrobenzyl, aminobenzyl, diphenylmethyl, phthalidyl or phenacyl), substituted alkyl esters (e.g. esters of trichloroethyl, t-butyl or allyl), aryl esters (e.g. esters of pentachlorophenyl or indanyl), esters with N-hydroxyamino compounds (e.g. esters with acetone oxime, acetophenone oxime, acetaldoxime, N-hydroxy succinimide or N-hydroxyphthalimide), or acid anhydrides with carbonic or carboxyl acids. Substituted amides and substituted hydrazides having high reactivity are also included in the term "carboxyl-protecting group". These protecting groups may carry the various substituents previously described.

Especially useful carboxylic acid derivatives of formula (I) are pharmacologically active esters suitable for pharmaceutical or veterinary use. The term "pharmacologically active esters" used herein refers to those showing strong antimicrobial activity when administered orally or parenterally. Such esters are exemplified by substituted alkyl esters (e.g. alkanoyloxyalkyl esters, alkoxyformyloxy alkyl esters or 2-oxo-1,3-dioxolenylmethyl esters), substituted aralkyl esters (e.g. phenacyl ester or phthalidyl ester), and substituted aryl esters (e.g. phenyl ester, xylyl ester or indanyl ester).

The term "light metal" denotes a metal belonging to the second to fourth period of groups I to III in the periodic table and which provides a physiologically acceptable ion in body fluids. Lithium, sodium, potassium,

magnesium, calcium and aluminium are representative of such "light metals".

The compounds (I) of the present invention can be prepared, for example, in accordance with the following reaction scheme:

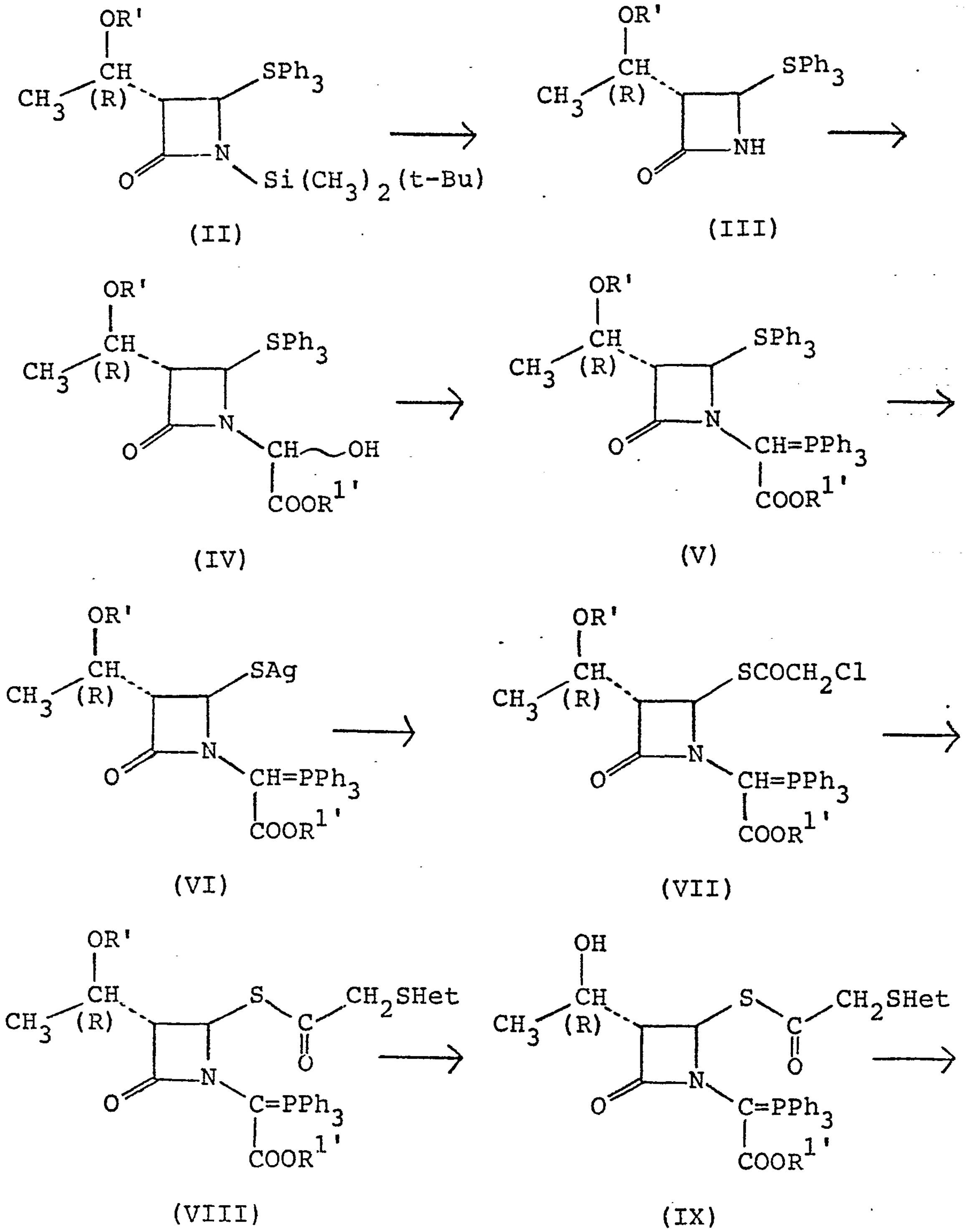

(II) (III)

(IV) (V)

(VI) (VII)

(VIII) (IX)

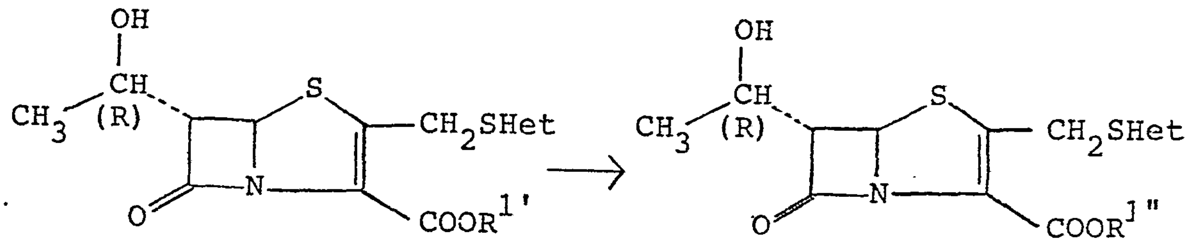

(I) (I)

wherein Ph is phenyl, R' is a hydroxy-protecting group, $R^{1'}$ is a carboxy-protecting group and $R^{1''}$ is hydrogen or a light metal.

In the above reaction scheme, the starting compounds of formula (II) are known _per se_ and disclosed, for example, in Japanese Patent Publication (Kokai) No. 25111/1975.

The compounds (I) of the present invention can also be prepared by conventional synthetic methods, for example, introduction of a heterocyclethio group, deprotection of a protecting group, conversion of a carboxylic acid to its salt, or esterification of a carboxylic acid.

Thus, the invention provides a process for preparing compounds (I) which comprises cyclizing a phosphine compound represented by the formula:

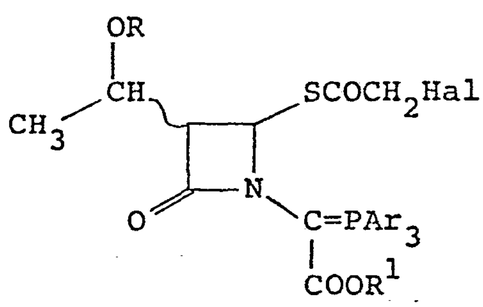

wherein Hal is halogen and Ar is aryl, or a reactive derivative thereof, by heating it and subjecting the resulting 6-(1-hydroxymethyl)-2-halomethyl-2-penem-3-carboxylic acid derivative of the formula:

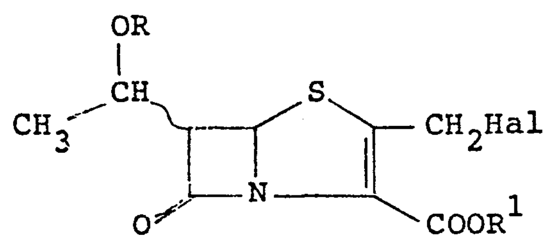

to reaction with a heterocyclic thiol of the formula:

HetSH,

or a reactive derivative thereof.

In carrying out the foregoing various reactions or in converting a given compound (I) to another compound (I), it may sometimes be necessary to protect reactive functional groups other than the reacting group involved in the intended reaction. For this purpose, a variety of conventional techniques for protection are all applicable to the processes of the invention. Such techniques are, for example, disclosed in the literature, such as J.F.W. McOmie Ed., "Protective Groups in Organic Chemistry", pp183, PLEUM Press, N.Y., 1973; S. Patai, Ed., "The Chemistry of Functional Groups", pp505, Interscience Publ., John Wiley & Sons Ltd. London, 1969; and Flynn Ed., "Cephalosporins and Penicillins", Academic Press, N.Y. 1972. Typical examples of the protection of reactive functional groups are acylation and etherification for a hydroxy group, acylation, enamination and silylation for an amino group, and esterification, amidation and acid anhydridation for a carboxylic acid.

It should be noted that "protection of a carboxylic acid" used herein also refers to esterification of the carboxylic acid at the 3-position for the purpose of obtaining a pharmacologically active ester. The esterification of a compound (I) having a free carboxylic acid can be carboxylate, and treating the latter with an acid halide having a proper ester residue.

The compounds of the formula (I) wherein $R^1$ is a carboxy-protecting group can be converted to compounds (I)

wherein $R^1$ is hydrogen according to any conventional deprotecting reaction, such as those described below.

In the following description, the carboxy-protecting group will sometimes be represented by a name corresponding to the group formed by the reaction between the carboxylic acid and the compound employed for protecting the carboxylic acid for the purposes of avoiding complexity of description. Thus, the protecting group, "$R^1$" contained in the moiety of the formula:

$$-COOR^1$$

will be referred to as an "ester" group for convenience.

a) The compounds (I) having highly reactive esters, amides, and anhydrides, can be deprotected by contact with an acid, a base, a buffer or an ion exchange resin in an aqueous solution. Less reactive protecting groups such as trichloroethyl, p-nitrobenzyl or phenacyl can be eliminated by treating with a combination of a metal and an acid or with a dithionate, or by a catalytic reduction.

b) Aralkyl esters can be eliminated by a hydrogenation using, e.g. palladium or nickel as a catalyst.

c) Aralkyl esters, cyclopropylmethyl esters and sulfonylethyl esters can be eliminated by solvolysis using a mineral acid, a Lewis acid, a sulfonic acid, or a strong carboxylic acid, if necessary, in the presence of a cation scavenger.

d) Phenacyl esters, alkenyl esters and hydroxyaralkyl esters can be eliminated using a base or a nucleophilic reagent. Photochemically active phenacyl esters can be eliminated by light radiation.

e) 2-Alkynyl esters can be converted to alkali metal salts be reaction with an alkali metal alkanoate and palladium triphenylphosphine.

f) Other conventional processes known for deprotecting carboxy-protecting groups can be employed in the present invention.

The compounds (I) are valuable antibiotics against various Gram positive and negative bacteria, and useful as drugs for human and veterinary purposes. They can be used for treating or preventing infections caused by Gram positive bacteria (e.g. Staphylococcus aureus, Streptococcus pyogenes, Bacillus subtilis, Bacillus cereus, Diplococcus pneumoniae or Corynebacterium diphtheriae) and Gram negative bacteria (e.g. Escherichia coli, Klebsiella pneumoniae, Proteus mirabilis, Proteus vulgaris, Proteus rettgeri, Proteus morganii, Enterobacter cloacae, Shigella sonnei, Salmonella paratyphi, Salmonella typhi or Serratia marsescens), including anaerobic bacteria (e.g. Bacteroides fragilis or Eubacterium lentum). The compounds can also be used as disinfectants, for preventing decay in perishables, as additives to foodstuffs, or in preventing bacterial growth in hygienic materials.

The invention includes a pharmaceutical or veterinary formulation comprising a compound (I) formulated for pharmaceutical or veterinary use, respectively.

The compounds (I) can be used in a wide variety of oral or parenteral dosage forms solely or in admixture with other coacting substances. Thus, pharmaceutical or veterinary formulations may be a mixture of 0.01 to 99% of a compound (I) with a pharmaceutically or veterinarily acceptable carrier which can be a solid material or liquid material in which the compounds are dissolved, dispersed or suspended. Formulations can be in unit dosage form. Solid formulations can take the form of e.g. tablets, powder, dry syrup, troches, granules, capsules, pills or suppositories. The liquid compositions can take the form of e.g. injections, ointments, dispersions, inhalants, suspensions, solutions, emulsions, syrups or elixirs. They may be flavored or colored, and tablets, granules and capsules may be coated.

Provided they do not exert an adverse effect on

compounds (I) the following, for example, may be used in formulations: diluents (e.g. starch, sucrose, lactose, calcium carbonate or kaolin); bulking agents (e.g. lactose, sugar, salt, glycine, starch, calcium carbonate, calcium phosphate, kaolin, bentonite, talc or sorbitol); binders (e.g. starch, acacia, gelatin, glucose, sodium alginate, tragacanth, carboxymethylcellulose, syrup, sorbitol or polyvinylpyrrolidone); disintegrators (e.g. starch, agar, carbonates or sodium laurylsulfate); lubricants (e.g. stearic acid, talc, paraffin, boric acid, silica, sodium benzonate, polyethylene glycol, cacao oil or magnesium stearate); emulsifying agents (e.g. lecithin, sorbitan monooleate, glycerin dioctanoate or acacia); suspending agents (e.g. sorbitol, methyl cellulose, glucose, sugar syrup, gelatin, hydroxyethylcellulose, carboxymethylcellulose, aluminium stearate gel or hydrogenated fats); solvents (e.g. water, buffer, peanut oil, sesame oil or methyl oleate); preservatives (e.g. methyl or ethyl p-hydroxybenzoate or sorbic acid); edible coloring agents, aromatic substances, solubilizing agents, buffers, stabilizing agents, analgesics, dispersing agents, wetting agents or antioxidants. These and other desired ingredients may be employed according to methods conventional in the art.

The compounds (I) having a carboxylic acid salt group are soluble in water, and may be conventionally used in solution for oral administration or for intravenous, intramuscular, or subcutaneous injection according to conventional methods.

The compounds (I) can be dissolved in aqueous or oily solvents for injection to give solutions in ampoules, but generally, more prolonged storage is possible by making vial preparations containing crystals, powder, microcrystals or lyophilizate of compounds (I), and dissolving or suspending the drug before use with a solvent for injection.

The vial preparation or injection can be given to a patient at a daily dose of e.g. 0.2 to 5 g depending upon the infecting bacteria, the condition of the patient and the interval of administration.

The compounds (I) in the form of pharmaceutically or veterinarily acceptable esters(e.g. indanyl, acetoxymethyl, pivaloyloxymethyl, ethoxycarbonyloxyethyl, phenacyl, phthalidyl, phenyl, tolyl, xylyl or methoxyphenyl esters) can be adsorbed through the digestive organs to some extent, and can be administered orally to human or veterinary subjects e.g. as powders, tablets, granules, capsules, dry syrups, emulsions, solutions or suspensions. They may be administered as pure compounds or as compositions comprising compounds (I) and carriers. The preparations can be made according to methods conventional in the art, and can be administered to patients at a daily dose of e.g. 1 to 2 g depending upon the condition of the patient and the disease in question.

This invention also provides a method for treating or preventing human or veterinary bacterial infections by administering to the human or animal subject a compound (I), e.g. at a daily dose of e.g. 0.2 to 5 g for injection, or e.g. 1 to 2 g for oral administration, or 10 μg to 1 mg for topical application, at intervals of e.g. 3 to 12 hours. The invention further includes compounds (I) for such use.

The above method is applicable for treating or preventing diseases caused by bacteria sensitive to compounds (I), e.g. pneumonia, bronchitis, pheumonitis, empyema, nasopharyngitis, tonsillitis, rhinitis, dermatitis, pustulosis, ulceration, abscess, wound and soft tissue infections, ear infections, osteomyelitis, septicemia, gastroenteritis, enteritis, respiratory or urinary tract infections, and pyelonephritis when caused by bacteria sensitive to compounds (I).

Preferably, the compounds (I) are given to patients

in the form of pharmaceutical or veterinary (as appropriate) preparations, e.g. powder, dry syrup, tablets, troches, granules, capsules, pills, suppositories, injections, ointments, dispersions, inhalants, suspensions, solutions, emulsions, syrups or elixirs. They may be in unit dosage form, e.g. tablets, troches, capsules, injections, vials, granules or powder in a separate container or package.

All of the pharmaceutical or veterinary preparations of the invention can be prepared by conventional methods familiar to those skilled in the art.

It will be readily understood by those skilled in the art that compounds (I) can also be used as germicides or antiseptics. In addition, they are useful as starting materials for preparing other compounds of the formula (I) and as antibiotic agents for testing the sensitivity of microorganisms.

Preferred and illustrative embodiments of the invention a given in the following Examples, wherein Infra Red (IR) and Nuclear Magnetic Resonance (NMR) data are shown by $\nu(cm^{-1})$ and $\delta(ppm)$ values (coupling constant J in Hz), respectively, and the following abbreviations are employed: Bu(=butyl), Ph(=phenyl), PMB(=p-methoxybenzyl), mM(=millimole).

Example 1 p-Methoxybenzyl 2-[(1-methyltetrazol-5-yl)-thiomethyl]-6-(R)-(1-hydroxyethyl)-penem-3-carboxylate [I-1].

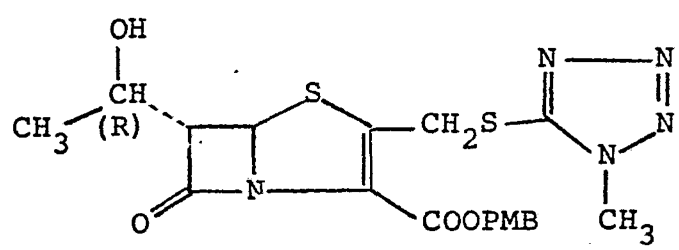

[I-1]

1) Preparation of compound [3].

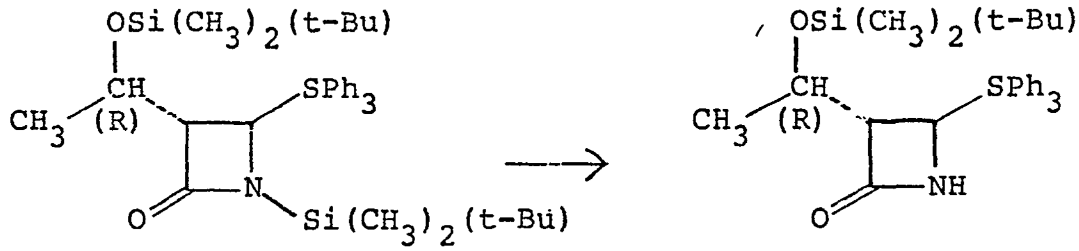

[2] [3]

Compound [2] (1.2g) is dissolved in tetrahydrofuran (THF) (10ml). To the solution are added acetic acid (0.14ml) and tetraethylammonium fluoride (0.45g), and the resulting mixture is stirred for 30 minutes at room temperature. The reaction mixture is diluted with ethyl acetate (100ml) and worked up in a conventional manner to give the desired N-desilyl compound [3] (1.1g).

IR($CHCl_3$): 3390, 2950, 2925, 1760$cm^{-1}$.

NMR($CDCl_3$): $\sim$0.05(s,6H), 0.72(s,9H), 1.25(d,J=7,3H), 3.11(t,J=2.5,1H), 4.23(dq,J=25 and 7,1H), 4.43(brs,1H), 4.55(d,J=3,1H), 7.2-7.7(m,J=15).

2) Preparation of compound [4].

[3] 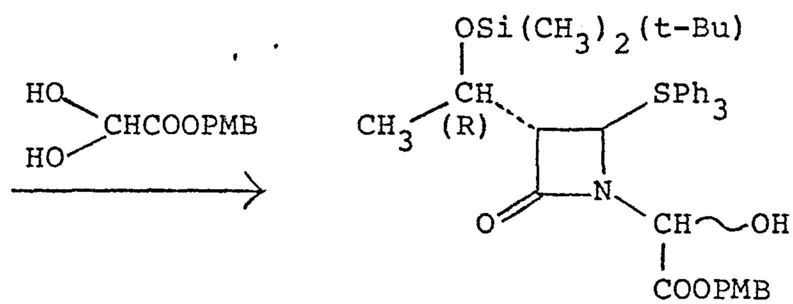

[4]

To a mixture of the N-desilyl compound [3] (1.1g) and p-methoxybenzyl glyoxylate hydrate (0.51g) in THF (14ml) is added triethylamine (0.28ml), and the mixture is left overnight at room temperature. The reaction mixture is

concentrated *in vacuo* to give the crude product [4] (1.5g), which is used in the following reaction without purification.

The product obtained above consists of two stereo isomers having different configurations to each other with respect to the hydroxy group at α-position. The isomers can be separated by chromatography over silica gel using benzene/ethyl acetate=9/1 as an eluent. The isomer contained in the first eluate is designated as isomer (a), and the other as isomer (b).

IR($CHCl_3$): 3400, 2950, 2925, 1765, 1500(sh)$cm^{-1}$.

NMR($CDCl_3$) of [4a]: 0.07(s,6H), 0.72(s,9H), 0.96(d,J=6,3H), 3.15-3.25(m,1H), 3.80(s,3H), 3.9-4.3(m,1H), 4.19(s,1H), 4.47(d,J=3,1H), 5.09(d,J=12) and 5.32(d,J=12)[ABq,2H], 6.87(d,J=9)[ABq,2H part of 4H], 7.2-7.6(m,25H).

NMR($CDCl_3$) of [4b]: 0.07(s,6H), 0.72(s,9H), 0.91(d,J=6,3H), 3.15-3.25(m,1H), 3.80(s,3H), 3.9-4.3(m,1H), 4.79(s,1H), 4.40(d,J=2.5,1H), 4.90(d,J=12) and 5.15(d,J=12)[ABq,2H], 6.94(d,J=9)[ABq,2H part of 4H], 7.2-7.6(m,25H).

3) Preparation of compound [5].

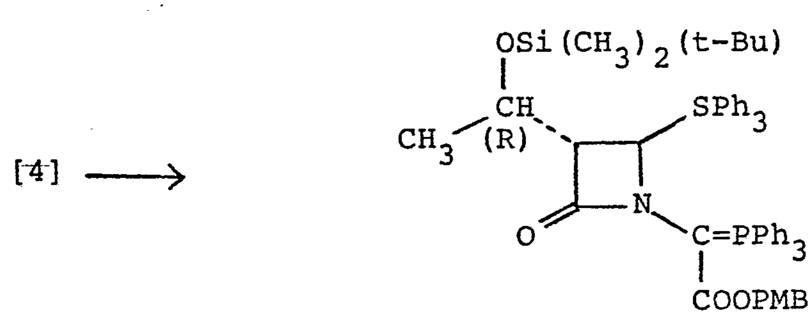

[5]

The compound [4] (1.5g) is dissolved in THF (30ml). To the solution is added 2,6-lutidine (0.46ml) and the resulting mixture is cooled to -30°C. After addition of thionylbromide (0.23ml), the reaction mixture is stirred for 30 minutes and then allowed to warm up to 0°C, and stirring is continued for 10 minutes. The reaction mixture is diluted with ethyl acetate (100ml) and washed successively with water, an aqueous $NaHCO_3$ solution and brine. The mixture is evaporated *in vacuo* and the resulting residue is dissolved in THF (20ml). To the THF solution are added triphenylphosphine (0.79g) and 2,6-lutidine (0.35ml) and the mixture is left overnight at room temperature. The reaction mixture is worked up in a conventional manner and purified by chromatography over silica gel using n-hexan/ethyl acetate=4/1 and benzene/ethyl acetate=4/1 as the eluents. The ylide compound [5] (1.66g) is thus obtained.

IR ($CHCl_3$): 2950, 2925, 1745, 1610$cm^{-1}$.

NMR ($CDCl_3$): 0.74 and 0.80 (s x 2,9H), 3.77 (s,3H), 6.6-8.0 (m,34H), and other peaks which cannot be assigned.

4) Preparation of compound [6].

[5]

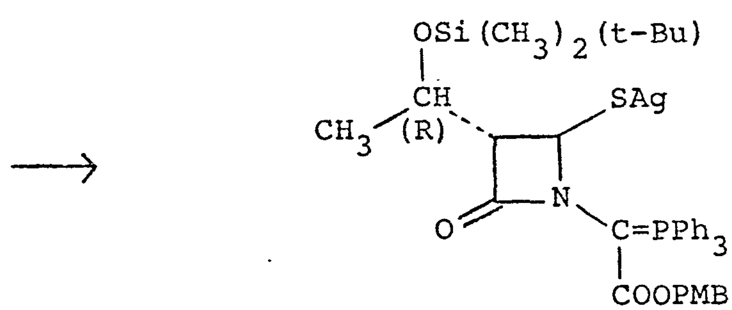

[6]

The ylide compound [5] (0.9g) is dissolved in methylene chloride (5ml) and the resulting solution is

diluted with methanol. To the solution are added a $AgNO_3$(195mg) solution in methanol(6.5ml) and pyridine(0.09ml), and the mixture is stirred for 1 hour at room temperature. After concentration of the reaction mixture *in vacuo*, the residue is diluted with methylene chloride, washed with water, dried, and worked up in a conventional manner to give the silver salt [6](0.87g).

IR($CHCl_3$): 3410, 2950(sh), 2925, 1740, 1615cm$^{-1}$.

5) Preparation of compound [7].

[6]

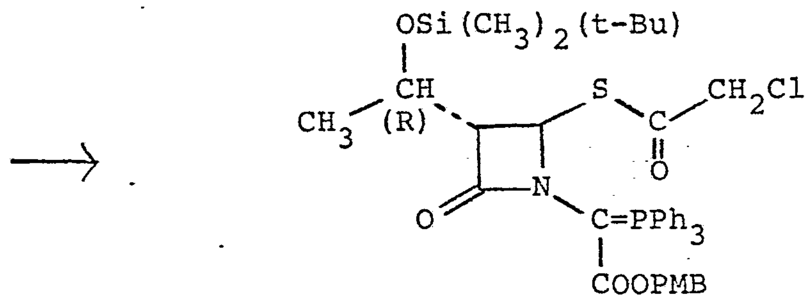

[7]

The compound [6](0.87g) is dissolved in methylene chloride(14ml). The solution is cooled to -40°C, and pyridine(0.44ml) and chloroacetyl chloride(0.36ml) are adde thereto. The mixture is stirred for 15 minutes and then diluted with ethyl acetate(100ml) and water(10ml). Precipitated insoluble substance is filtered off with sellaite. The filtrate is evaporated *in vacuo* and the resultar oily residue is purified by chromatography over silica gel using benzene/ethyl acetate(=4/1, and then 2/1) as eluents to give the keto-ylide compound [7](0.48g).

IR($CHCl_3$): 2950, 2925, 1750, 1690(w), 1610cm$^{-1}$.

6) Preparation of compound [VIII-1].

[7] 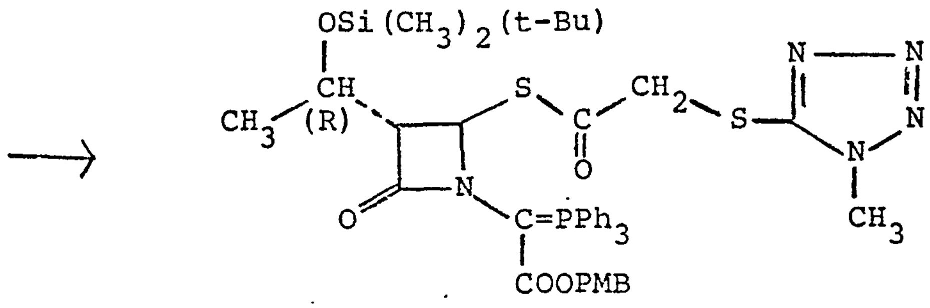

[VIII-1]

To the keto-ylide compound [7](310mg) dissolved in methylene chloride(6ml) are added sodium 1-methyltetrazolethiolate(210mg), tetra-n-butylammonium bromide(66mg) and water(0.2ml). The mixture is stirred vigorously for 2 hours at room temperature. The reaction mixture is diluted with ethyl acetate and worked up in a conventional manner to obtain the crude product [VIII-1].

IR($CHCl_3$): 1755, 1690(w), 1610$cm^{-1}$.

7) Preparation of compound [IX-1].

[VIII-1] 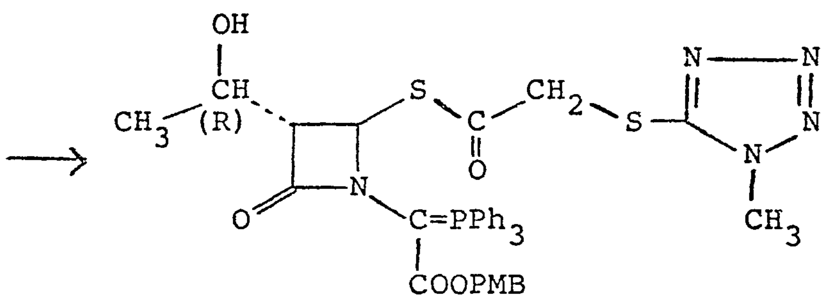

[IX-1]

The compound [VIII-1] obtained above is dissolved in acetonitrile(1.1ml), and the resultant solution is diluted with methanol(5ml). The solution is then added with 1N HCl(4ml) and stirred for 1 hour at room temperature. The reaction mixture is worked up in a conventional manner to yield an oily product which is chromatographed over silica

gel using methylene chloride/acetonitrile=1/1 as an eluent to give the compound [IX-1] (170mg).

IR($CHCl_3$): 3500, 1760, 1610$cm^{-1}$.

NMR($CDCl_3$): 3.78 (brs,3H), 3.95 (s,3H), 4.2-4.4 (m,2H), 7.3-7.9 (m,15H), and other peaks which cannot be assigned.

8) Preparation of the ultimate compound [I-1].

The compound [IX-1] (158mg) is dissolved in methylene chloride (0.5ml). The solution is diluted with benzene (11ml) and heated at 75°C for 4.5 hours. After completion of the reaction, the solvent is evaporated off <u>in vacuo</u> and the resulting residue is purified by chromatography over silica gel using methylene chloride/acetonitrile=1/1 as an eluent to give the ultimate penem compound [I-1] (75mg).

IR($CHCl_3$): 1790, 1705, 1610, 1580, 1320$cm^{-1}$.

NMR($CDCl_3$): 1.29 (d,J=6,3H), 2.72 (m,1H(-OH)), 3.68 (d,J=1.5) [d,d,half of 1H($H_6$)], 3.78 (s,3H) [and half of $H_6$], 3.70 (s,3H), 4.0-4.4 (m,1H), 4.41 (d)+4.77 (d) [ABq,J=15, 2H], 5.18 (s,2H), 5.57 (d,J=1.5,1H), 6.86 (d)+7.35 (d) [ABq,J=9, 4H].

<u>Example 2</u> Sodium 2-[(1-methyltetrazol-5-yl)thiomethyl]-6-(R)-(1-hydroxyethyl)-penem-3-carboxylate [I-2].

[I-1]

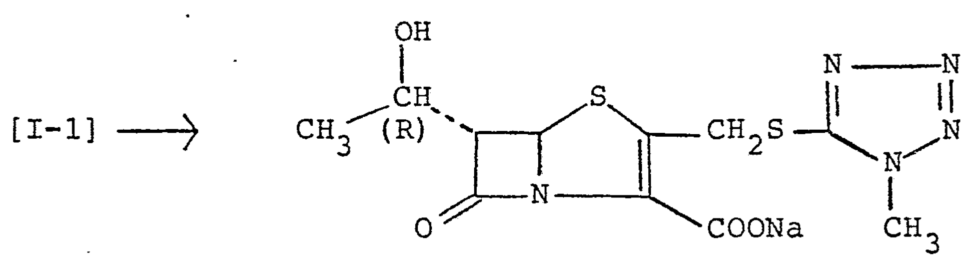

[I-2]

The crude penem product [I-1] contaminated with triphenylphosphine oxide as a by-product, which was prepared from the compound [IX-1] (74mg, 0.1mM) according to the process of Example 1-8), is dissolved in anisole/methylene chloride (1ml/0.1ml). The solution is added to aluminum chloride (53mg) in anisole/methylene chloride (1ml/0.1ml) at -50°C and the resulting mixture is stirred for 1 hour. To the reaction mixture are added a $NaHCO_3$-phosphate buffer (pH 7) and methylene chloride, and the resulting mixture is stirred vigorously. After insoluble substances are filtered off, the aqueous layer is separated and washed with methylene chloride. The aqueous solution is subsequently desalted by passing through a Diaion HP-20 column (=Diaion HP-20=trade name of styrene divinylbenzene copolymer produced by Mitsubishi Kasei Co., Ltd., Japan). The fractions eluted with 5% methanol are collected and lyophilized. The sodium salt [I-2] (13mg) is thus obtained as white powder.

UV ($\lambda_{max}^{H_2O}$): 255 (sh), 314nm.

Example 3 p-Methoxybenzyl 2-[(1-(2-hydroxyethyl)-tetrazol-5-yl)-thiomethyl]-6-(R)-(1-hydroxyethyl)-penem-3-carboxylate [I-3].

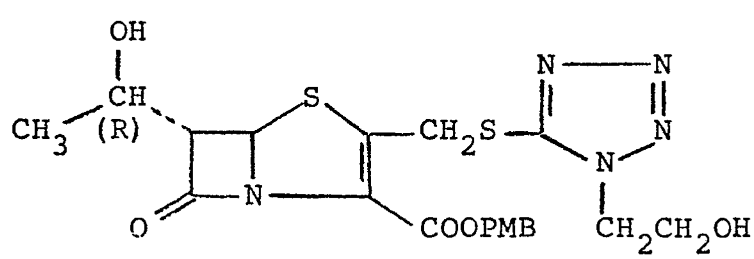

[I-3]

1) Preparation of compound [VIII-2].

[7]

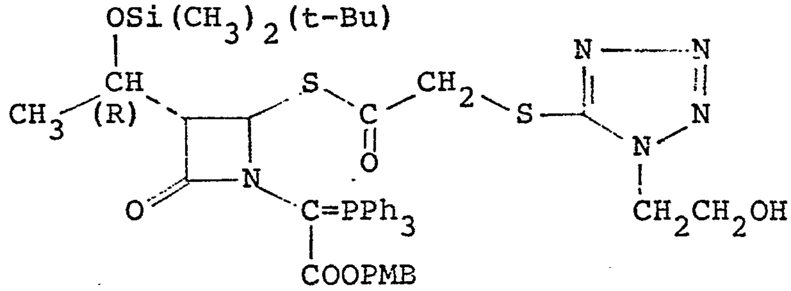

[VIII-2]

The compound [VIII-2] is prepared from the compound [7] in substantial accordance with the teaching of Example 1-6) except that 4 equivalents of sodium 1-(2-hydroxyethyl)-tetrazolethiolate and 1 equivalent of tetra-n-butylammonium bromide are used and that the reaction mixture is stirred for 6 hours at room temperature.

IR($CHCl_3$): 3350(br), 2950, 2925, 2850, 1750, 1690, 1615$cm^{-1}$.

2) Preparation of the ultimate compound [I-3].

The dimethyl-t-butylsilyl group of the compound [VIII-2] is removed in substantial accordance with the teaching of Example 1-7) to yield the corresponding hydroxy compound.

IR($CHCl_3$): 3370, 2975, 1755, 1680, 1610$cm^{-1}$.

The ultimate compound [I-3] is prepared from the hydroxy compound obtained above in substantial accordance with the teaching of Example 1-8) except that the reaction is carried out at 80°C for 3 hours.

IR($CHCl_3$): 3400, 2950, 1790, 1700, 1610, 1575$cm^{-1}$.

NMR($CDCl_3$): 1.28(d,J=6,3H), 2.6-3.3(m,2H), 3.72(dd, J=7 and 1, 1H), 3.78(s,3H), 3.9-4.5(m,5H), 4.42(d)

+ 4.73(d)[ABq,J=14,2H], 5.16(s,2H), 5.56(d,J=1,1H), 6.87(d) + 7.35(d)[ABq,J=9,4H).

Example 4  Sodium 2-[(1-(2-hydroxyethyl)tetrazol-5-yl)-thiomethyl]-6-(R)-(1-hydroxyethyl)-penem-3-carboxylate [I-4].

[I-3]

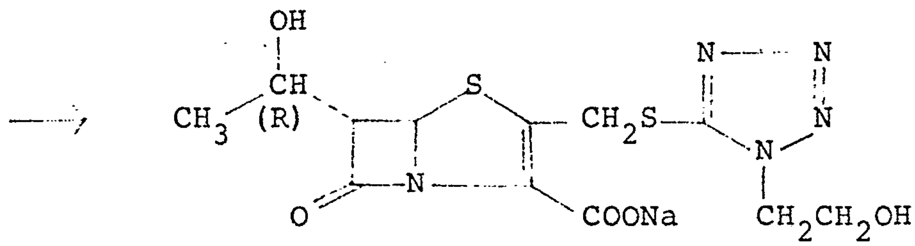

[I-4]

The desired compound [I-4] is obtained in substantial accordance with the teaching of Example 2, except that water is used rather than 5% aqueous methanol and that the compound [I-3] is reacted with 5 equivalents of anhydrous aluminum chloride.

UV($\lambda_{max}^{H_2O}$): 253(sh), 315nm.

NMR($H_2O$): 1.73(d,J=6,3H), 4.32(dd,J=6 and 1.5,1H), 4.47(t,J=6,2H), 4.67(q,J=6,1H), 5.0-5.2(m,4H), 6.04(d,J=1.5,1H).

Example 5  p-Methoxybenzyl 2-[(1-aminotetrazol-5-yl)-thiomethyl]-6-(R)-(1-hydroxyethyl)-penem-3-carboxylate [I-5].

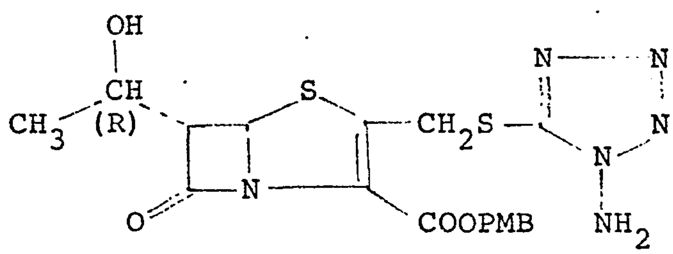

[I-5]

1) Preparation of compound [VIII-3].

[7] 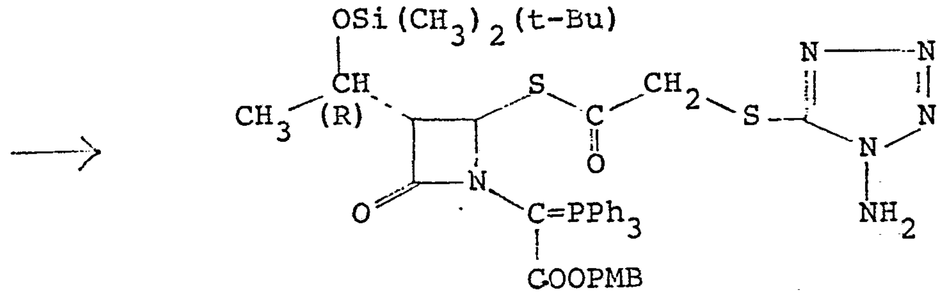

[VIII-3]

The compound [VIII-3] is prepared from the compound [7] in substantial accordance with the teaching of Example 1-6) except that 4 equivalents of sodium 1-(2-amino)-tetrazole mercaptide and 1 equivalent of tetra-n-butylammonium bromide are used and that the reaction mixture is stirred for 4.5 hours at room temperature.

IR($CHCl_3$): 3330, 2945, 2920, 2840, 1750, 1685, 1610$cm^{-1}$.

2) Preparation of the desired compound [I-5].

The dimethyl-t-butylsilyl group of the compound [VIII-3] is removed in substantial accordance with the teaching of Example 1-7). The desired compound [I-5] is obtained from the resultant hydroxy compound in substantial accordance with the teaching of Example 1-8) except that the reaction is carried out at 75°C for 3.5 hours.

<u>Example 6</u> Sodium 2-[(1-aminotetrazol-5-yl)-thiomethyl]-6-(R)-(1-hydroxyethyl)-penem-3-carboxylate [I-6].

[I-5] 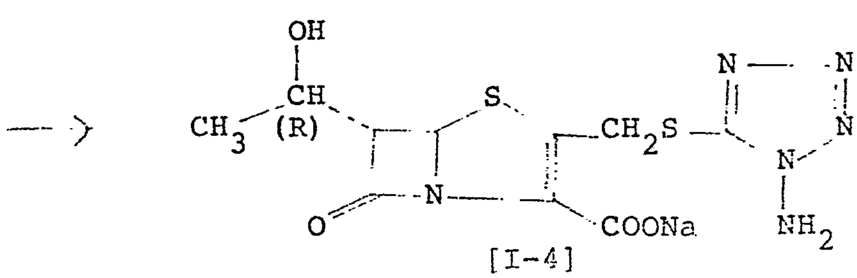

[I-4]

The desired compound [I-5] is obtained in substantial accordance with the teaching of Example 2 except that the compound [I-5] is reacted with 5 equivalents of anhydrous aluminum chloride.

UV ($\lambda_{max}^{H_2O}$): 264, 312nm.

NMR ($D_2O$, Ext.TMS): 1.69(d,J=6,3H), 4.31(dd,J=6 and 1.5,1H), 4.67(t,J=6,1H), 5.03(s,2H), 6.05(d,J=1.5,1H).

<u>Example 7</u> p-Methoxybenzyl 2-[(1-carbamoylmethyl-tetrazol-5-yl)-thiomethyl]-6-(R)-(1-hydroxyethyl)-penem-3-carboxylate [I-7].

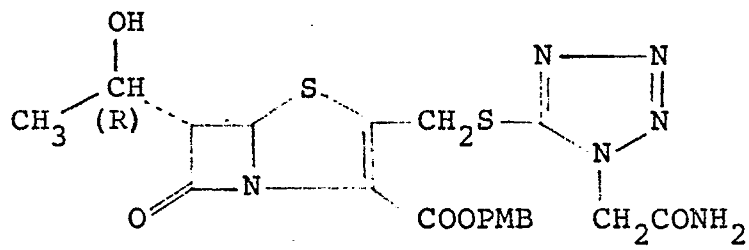

[I-7]

1) Preparation of compound [IX-2].

[7]

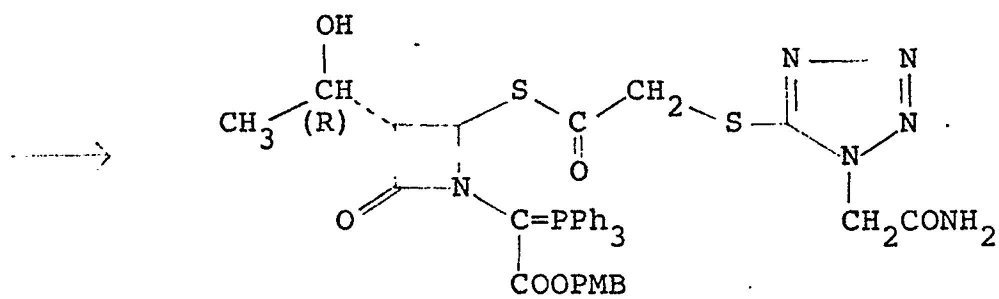

[IX-2]

The compound [VIII] wherein Het is carbamoyl-methyltetrazole is obtained from the compound [7] in substantial accordance with the teaching of Example 1-6) except that 4 equivalents of sodium 1-(carbamoylmethyl)-tetrazolethiolate and 1 equivalent of tetra-n-butylammonium bromide are used and that the reaction mixture is stirred

for 12 hours at room temperature. The dimethyl-t-butylsilyl group of the resulting compound is removed in substantial accordance with the teaching of Example 1-7) to provide the compound [IX-2].

IR($CHCl_3$): 3470, 3400, 3325, 3180, 2970, 1760, 1700, 1615$cm^{-1}$.

2) Preparation of the desired compound [I-7].

The desired compound [I-7] is obtained from the compound [IX-2] in substantial accordance with the teaching of Example 1-8) except that the reaction is carried out at 80°C for 4 hours.

IR(nujol): 3515, 1790, 1695, 1665$cm^{-1}$.

NMR($CDCl_3$+$CD_3CN$): 1.21(d,J=6,3H), 2.5-3.2(m,1H), 3.68(dd,J=6 and 1.5,1H), 3.76(s,3H), 4.07(q,J=6,1H), 4.40(d) + 4.72(d)[ABq,J=15,2H],4.98(s,2H), 5.14(s,2H), 5.54(d,J=1.5, 1H), 5.9-6.3(m,1H), 6.84(d) + 7.33(d)[ABq,J=9,4H).

<u>Example 8</u> Sodium 2-[(1-carbamoylmethyltetrazol-5-yl)-thiomethyl]-6-(R)-(1-hydroxyethyl)-penem-3-carboxylate [I-8].

[I-7]

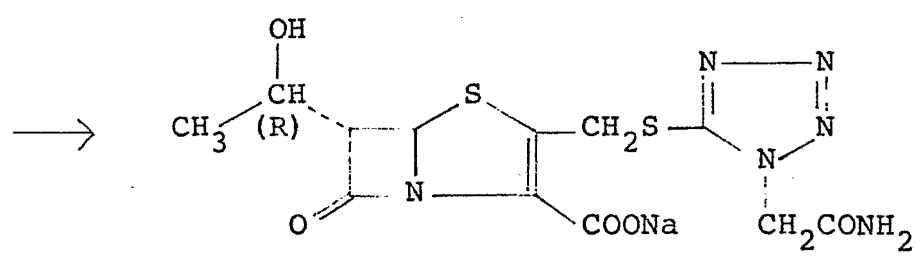

[I-8]

The desired compound [I-8] is obtained in substantial accordance with the teaching of Example 2, except that the compound [I-7] is reacted with 5 equivalents of anhydrous aluminum chloride.

UV($\lambda_{max}^{H_2O}$): 260, 315nm.

NMR($D_2O$): 1.73(d,J=6,3H), 4.31(dd,J=6 and 1.5,1H), 4.67(q,J=6,1H), 4.91(d) + 5.14(d)[ABq,J=14,2H], 5.82(s,2H), 6.04(d,J=1.5,1H).

Example 9 p-Methoxybenzyl 2-(1,3,4-thiadiazol-2-ylthiomethyl)-6-(R)-(1-hydroxyethyl)-penem-3-carboxylate [I-9].

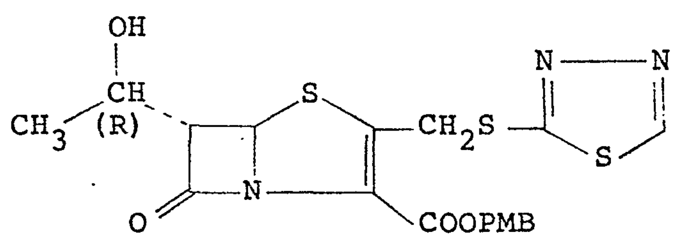

[I-9]

1) Preparation of compound [VIII-4].

[7] 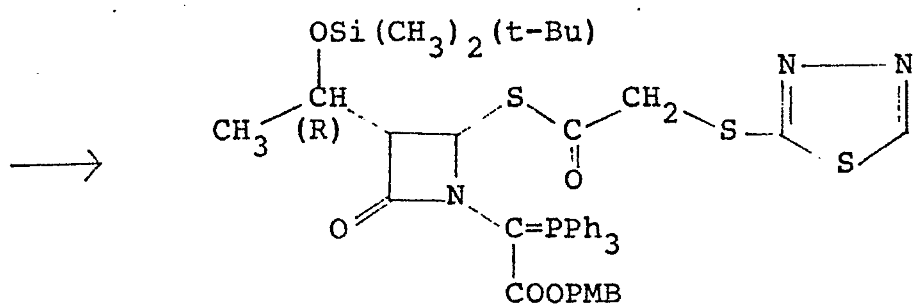

[VIII-4]

The compound [VIII-4] is prepared in substantial accordance with the teaching of Example 1-6) except that 3 equivalents of sodium 1,3,4-thiadiazol-2-mercaptide and 0.5 equivalent of tetra-n-butylammonium bromide are used and that the reaction mixture is stirred for 3 hours at room temperature.

IR($CHCl_3$): 3400, 2945, 2920, 2840, 1750, 1685, 1610$cm^{-1}$.

2) Preparation of compound [IX-3].

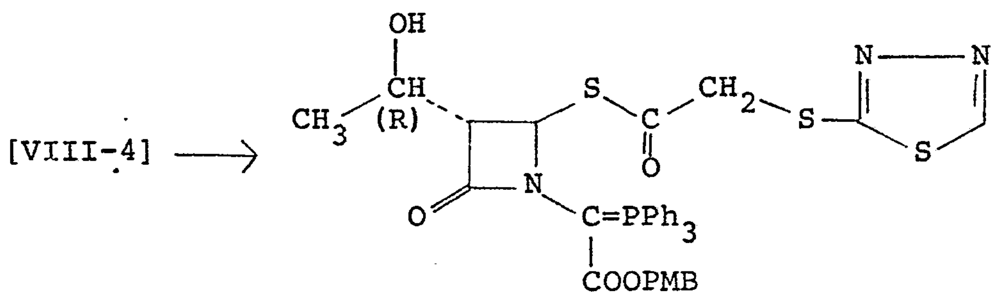

[IX-3]

The compound [IX-3] is obtained in substantial accordance with the teaching of Example 1-7) except that the compound [VIII-4] is used as a starting compound.

IR($CHCl_3$): 3420, 3000, 1755, 1680(sh), 1615cm$^{-1}$.

3) Preparation of the ultimate compound [I-9].

Using the compound [IX-3] as a starting compound, the desired compound [I-9] is obtained in substantial accordance with the teaching of Example 1-8) except that the reaction is carried out at 80°C for 4 hours.

IR($CHCl_3$): 3515, 1790, 1695, 1665cm$^{-1}$.

NMR($CDCl_3$+$CD_3CN$): 1.21(d,J=6,3H), 2.5-3.2(m,1H), 3.68(dd,J=6 and 1.5,1H), 3.76(s,3H), 4.07(q,J=6,1H), 4.40(d) + 4.72(d)[ABq,J=15,2H],4.98(s,2H), 5.14(s,2H), 5.54(d,J=1.5, 1H), 5.9-6.3(m,1H), 6.84(d) + 7.33(d)[ABq,J=9,4H].

Example 10 Sodium 2-(1,3,4-thiadiazol-2-yl-thiomethyl)-6-(R)-(1-hydroxyethyl)-penem-3-carboxylate [I-10].

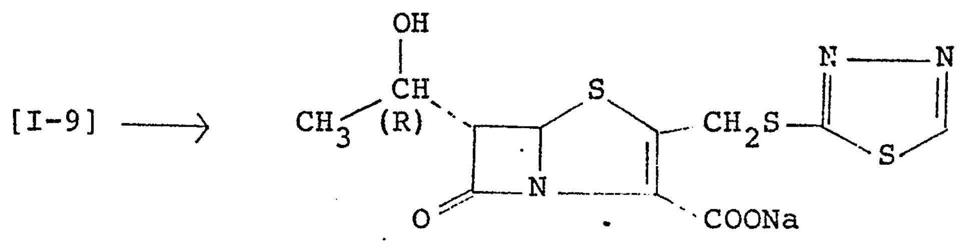

[I-10]

The compound [I-10] is obtained in substantial accordance with the teaching of Example 2 except that the compound [I-9] is reacted with 5 equivalents of anhydrous aluminum chloride.

UV($\lambda_{max}^{H_2O}$): 257, 313nm.

NMR($D_2O$): 1.72(d,J=6,3H), 4.28(dd,J=6 and 1.5,1H), 4.67(q,J=6,1H), 5.10(s,2H), 6.02(d,J=1.5,1H).

Example 11

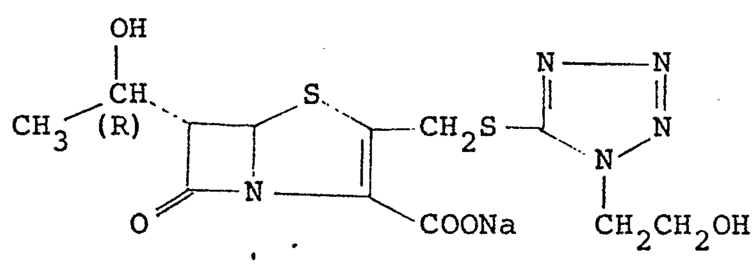

[I-4]

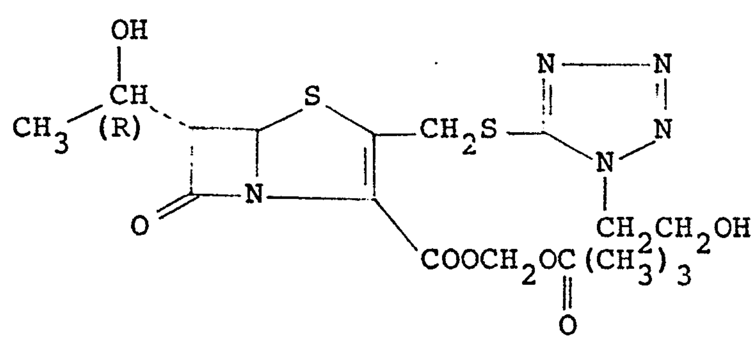

[I-11]

1) 2-[(1-(2-Hydroxyethyl)tetrazol-5-yl)-thiomethyl]-6-(R)-(1-hydroxyethyl)-penem-3-carboxylate

[I-4](imM) is dissolved in 2 - 5 parts by weight of N,N-dimethylformamide. To the solution is added 1 - 2 equivalents of iodomethyl pivalate while cooling at -20°C, and the resulting mixture is stirred for 1/4 - 2 hours. The reaction mixture is diluted with ethyl acetate and washed with ice water and aqueous $NaHCO_3$ solution, dried and concentrated _in vacuo_. The resulting residue is crystallized from ethyl acetate to give the pivaloyloxymethyl ester of the starting carboxylic acid [I-11].

UV($\lambda_{max}^{95\% EtOH}$): 257( : 5300), 335( : 6100)nm.

IR($CHCl_3$): 3380(br), 2970, 1790, 1750, 1730(sh)$cm^{-1}$.

NMR($CDCl_3$): 1.21(s,9H), 1.30(d,J=6,2H), 2.6-3.2(m), 3.74(dd,J=1.5 and 6.5,1H), 4.0-4.5(m,5H), 4.45(d) + 4.76(d)(ABq,J=15,2H), 5.60(d,J=1.5,1H), 5.79(d) + 5.89(d)(ABq,J=5.5,2H).

2) The pivaloyloxymethyl ester [I-11] is also obtained when the potassium salt, instead of the sodium salt, is used in the above reaction.

3) The pivaloyloxymethyl ester (250mg), coan starch(150mg) and magnesium stealate(5mg) are admixed, granulated and filled in gelatin capsules in a conventional manner.

One to three capsules obtained above can be orally administered thrice a day to treat patients infected with _Staphylococcus aureus_.

CLAIMS:

1. A compound of the formula:

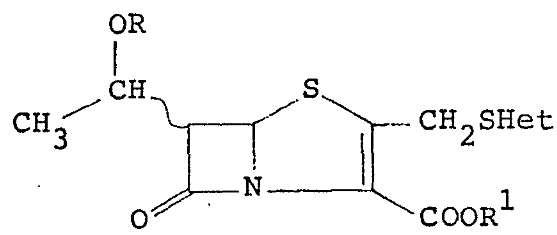

wherein R is hydrogen or a hydroxy-protecting group, $R^1$ is hydrogen, a light metal or a carboxy-protecting group, and Het is a five-membered heterocyclic group.

2. A compound as claimed in claim 1, wherein $R^1$ is hydrogen, sodium, potassium, calcium, acetoxymethyl, diphenylmethyl, 1-(ethoxycarbonyloxy)ethyl, indanyl, p-methoxybenzyl, phthalidyl or pivaloyloxymethyl.

3. A compound as claimed in claim 1 or claim 2, wherein Het is 1,2,4-thiadiazol-2-yl or tetrazol-5-yl optionally substituted by amino, $C_1$-$C_3$ alkyl, or $C_1$-$C_3$ alkyl substituted by carbamoyl, hydroxy or protected hydroxy.

4. A process for preparing a compound as claimed in any one of claims 1 to 3, which process comprises either (a) cyclizing a phosphine compound represented by the formula:

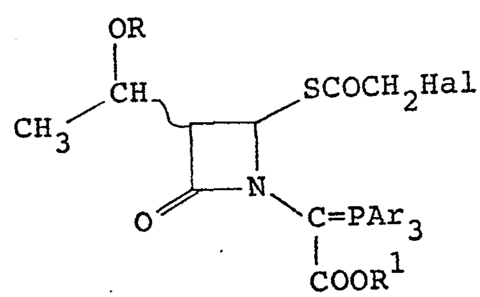

wherein Hal is halogen and Ar is aryl, or a reactive derivative thereof, by heating it and subjecting the resulting 6-(1-hydroxyethyl)-2-halomethyl-2-penem-3-carboxylic acid derivative of the formula:

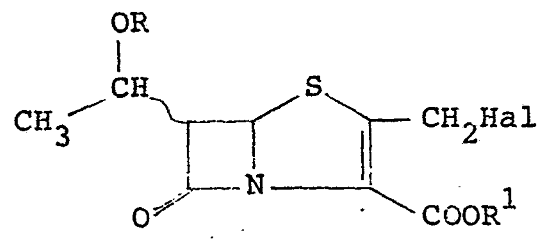

to reaction with a heterocyclic thiol of the formula:

HetSH,

or a reactive derivative thereof; or (b) preparing a compound as claimed in any one of claims 1 to 3 which is salt by neutralizing a compound of the formula:

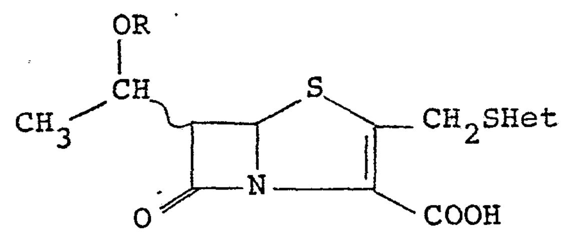

,

or a reactive derivative, with a base; or (c) preparing a compound as claimed in any one of claims 1 to 3 which is a carboxylic acid by deprotecting the carboxy group of a compound of the formula:

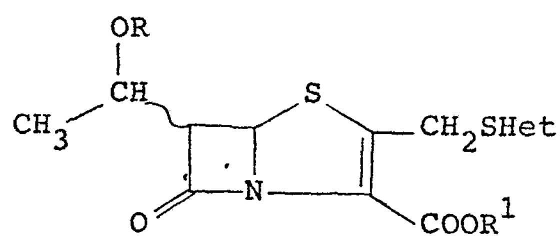

wherein $R^1$ is other than hydrogen.

5. A process as claimed in claim 4(c), wherein the starting compound is a carboxylic acid ester and deprotecting is achieved by hydrolysis thereof.

6. A process for preparing a compound as claimed in any one of claims 1 to 3, which process comprises the penultimate step in the following reaction sequence, optionally preceded by one or more of the earlier steps (in sequence if more than one such step), and optionally

followed by the final step:-

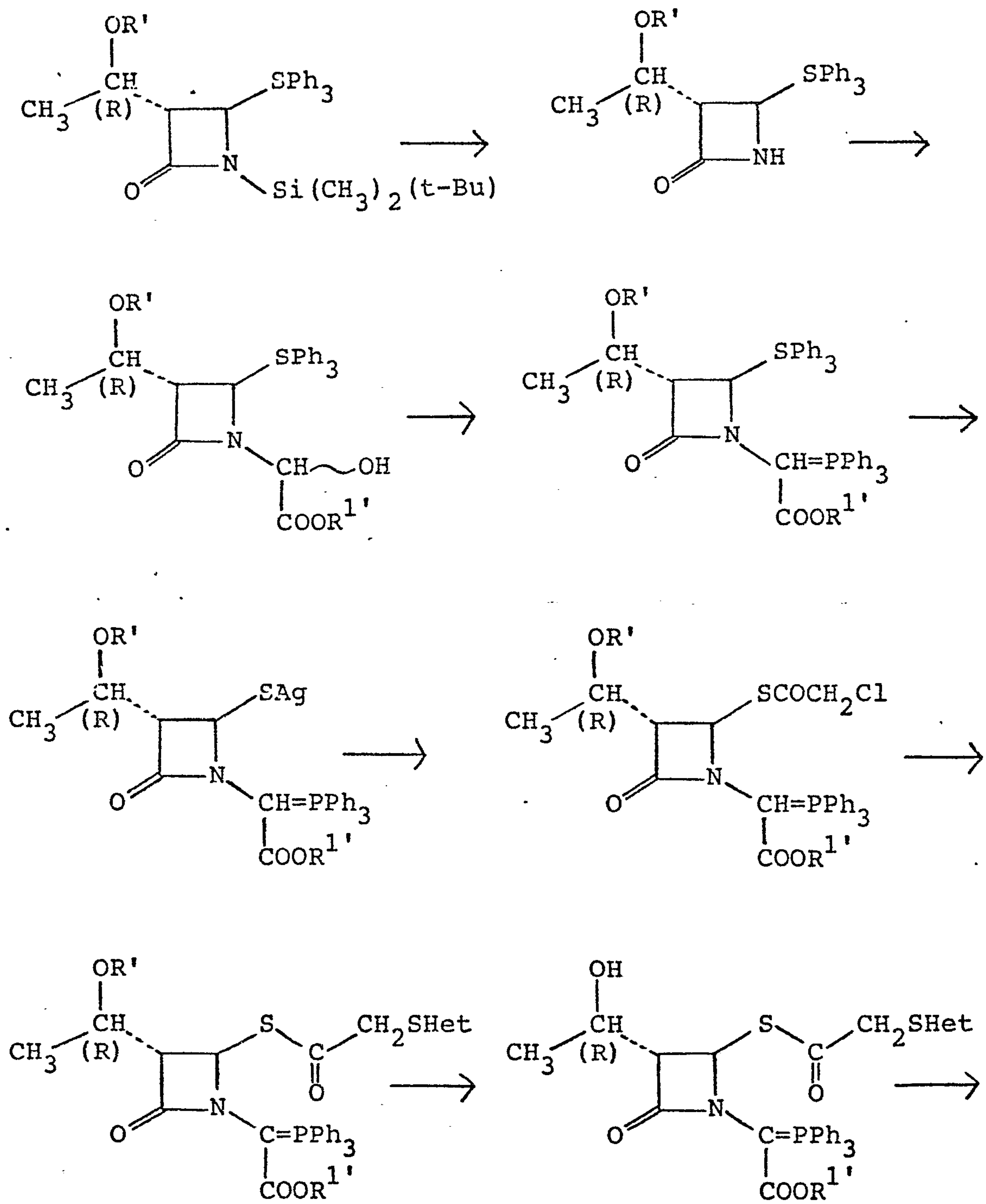

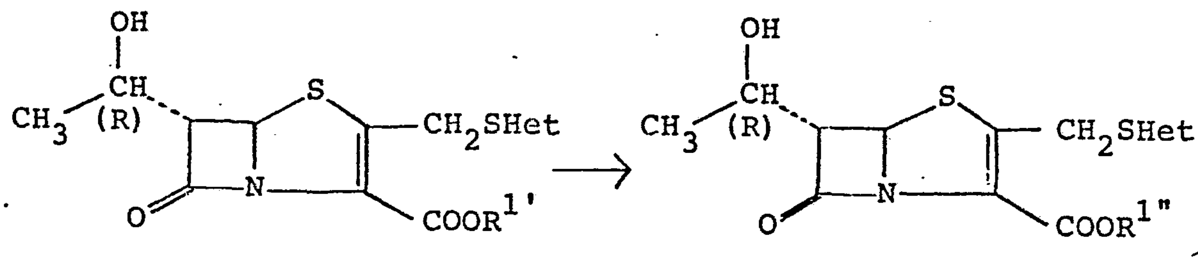
;

the compounds in the foregoing sequence optionally having a different steric configuration, and wherein Ph is phenyl, R' is a hydroxy-protecting group, $R^{1'}$ is a carboxy-protecting group and $R^{1''}$ is hydrogen or a light metal, or any chemical equivalent(s) thereof; or which process comprises any chemical equivalent of such step or steps.

7. A pharmaceutical or veterinary formulation which comprises a compound as claimed in any one of claims 1 to 3 formulated for pharmaceutical or veterinary use, respectively, and optionally in unit dosage form.

8. A formulation as claimed in claim 7 also comprising a pharmaceutically acceptable or veterinarily acceptable, respectively, diluent, carrier or excipient.

9. A method for combating bacteria in an environment or of inhibiting their appearance therein which comprises bringing an effective amount of a compound as claimed in any one of claims 1 to 3 into contact with the bacteria or environment.

10. A compound as claimed in any one of claims 1 to 3 or a formulation as claimed in claim 7 or claim 8 for use in a method as claimed in claim 9 or in the treatment or prevention of bacterial infection in a human being or other animal or as a disinfectant or for preserving a perishable article, substance or material from bacterial decay or as a foodstuff additive.

European Patent Office

# EUROPEAN SEARCH REPORT

0115969

Application number

EP 84 30 0849

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | EP-A-0 069 373 (TAKEDA)<br>* Pages 67-73, examples 3-10; claims * | 1-10 | C 07 D 499/00<br>A 61 K 31/43 //<br>C 07 F 7/18<br>C 07 F 9/65 |
| | --- | | |
| X | FR-A-2 449 690 (FARMITALIA CARLO ERBA)<br>* Pages 26-27, examples 29,30; page 38; claims * | 1-10 | |
| | --- | | |
| Y | EP-A-0 002 210 (MERCK)<br>* Claims * | 1-10 | |
| | --- | | |
| Y | EP-A-0 003 960 (CIBA-GEIGY)<br>* Claims * | 1-10 | |
| | --- | | |
| Y | GB-A-2 042 515 (BRISTOL-MYERS)<br>* Claims * | 1-10 | TECHNICAL FIELDS SEARCHED (Int. Cl. 3) |
| | --- | | |
| Y | CHEMICAL ABSTRACTS, vol. 98, no. 23, 6th June 1983, page 620, no. 197887x, Columbus, Ohio, US & JP - A - 57 179 190 (YAMANOUCHI PHARMACEUTICAL CO., LTD.) 04-11-1982 * Abstract * | 1-10 | C 07 D 499/00<br>A 61 K 31/00 |
| | --- | | |
| P,X | GB-A-2 118 181 (FARMITALIA CARLO ERBA)<br>* Claims * | 1-10 | |
| | ----- | | |

The present search report has been drawn up for all claims

| Place of search | Date of completion of the search | Examiner |
|---|---|---|
| THE HAGUE | 14-05-1984 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82